# EUROPEAN PATENT APPLICATION

(11) **EP 3 920 190 A1**
(43) Date of publication of application: **08.12.2021**
(21) Application number: 20178345.3
(22) Date of filing: 04.06.2020
(51) Int. Cl.: G16H 30/40, G16H 40/40

(54) **BIAS DETECTION IN SENSOR SIGNALS**

(71) Applicant: Koninklijke Philips N.V., 5656 AG Eindhoven (NL)
(72) Inventor: LAUTE, Niels, 5656 AE Eindhoven (NL); GEURTS, Lucas Jacobus Franciscus, 5656 AE Eindhoven (NL)
(74) Representative: Philips Intellectual Property & Standards

(57) **Abstract**

Some embodiments are directed to a bias-system for detecting bias in information from a sensor system. The bias-system may include identifying one or more of the participants in a sensor signal by matching a participant with a corresponding template, and identifying a bias in the multiple sensor signals using the identified participants.

## Description

### FIELD OF THE INVENTION

The presently disclosed subject matter relates to a bias-system for detecting bias in information from a sensor system, a machine learnable model, a computer implemented method for detecting bias in information from a sensor system, and a computer readable medium.

### BACKGROUND OF THE INVENTION

AI and machine learning technologies are improving rapidly. To train these systems, e.g., so they can support humans with certain tasks, large amounts of data is required. Ideally, these training data sets are complete and unbiased. However, research shows that this is often not the case: data sets can contain biases that can have a negative impact on the outcome and on the quality of the AI system.

A well-known example is the presence of a bias in current image data collections. Neural networks, e.g., Convolutional Neural Network (CNN) trained over such a training set can achieve a high quality, e.g., in object classification or image generation. However, an AI model, such as a neural network which is trained on such a training set will learn to reproduce the bias that is inherent in them. This will also affect the ability of the model to generalize, e.g., its effectiveness on new samples. In other words, the bias that is present in current training sets implies limits on the quality of a model that is trained on it. For example, the article "A Deeper Look at Dataset Bias", by Tatiana Tommasi, et al., included herein by reference, describes several problems that bias can have in the context of artificial intelligence. In particular, they found that attempts of undoing dataset bias with existing ad-hoc learning algorithms do not help and that bias will persist.

The inventors found that bias is also present in clinical datasets that may, e.g., by used to train AI models. These include, e.g., capture biases related to how the data was acquired, and category or label bias related to the selection of categories that was made for the model. Moreover, categories may be poorly defined, further contributing to category bias.

When the data reflects a human decision process, e.g., in healthcare, the data may also comprise human biases. For example, the data set may comprise clinical conclusions or decisions that were wrong. Training on wrong training material will inherently damage the capabilities of the trained model.

Training data sets for clinical decision support are often generated based on data that was created by humans: clinical data is combined with medical conclusions of a single clinician, or multiple clinicians, e.g., a multi-disciplinary tumorboard. The latter brings additional complexity: personnel relationships or organizational hierarchy can influence the decision-making and thus the quality of the training data set.

Key decisions in modern health care systems are often made by groups of people rather than individuals. For example, the article "Systematic biases in group decision-making: implications for patient safety", by Russell Mannion and Carl Thompson, included herein by reference, describes a study on group decision-making behavior in the healthcare system. In the article, theories are formed from organization studies and decision science to explore the ways in which patient safety may be undermined or threatened in health care contexts as a result of four systematic biases arising from group decision-making: 'groupthink', 'social loafing', 'group polarization' and 'escalation of commitment'.

### SUMMARY OF THE INVENTION

Several embodiments are provided to detect bias. In particular, detecting such bias may be used to improve machine learning on training sets in which such bias may be present. For example, it was an insight of the inventors that bias may be detected by utilizing not just the data in which the bias may be problematic but to use also additional data from which such bias may be deduced. For example, although bias may not be directly visible, say, from a radiology image, the bias may be derived from analyzing a discussion about such an image. For example, determining the participants to such a discussion may help reduce bias, since particular participants may opt more often for a particular course of action, may have some outcomes more often than others, may themselves be biased in some manner, e.g., when it comes to determining patient parameters, such as clinical segmentations, etc. Furthermore, in an embodiment, an understanding of group dynamics, internal relationships and/or loyalty in a team is used to flag biases in clinical multi-party decision making.

If a training dataset includes biased or corrupted data, the outcome of machine learnable models trained thereon will have unwanted biases embedded as well. This can eventually lead to wrong conclusions or decisions taken by or with a model trained on such data. In an embodiment, this problem is addressed, e.g., by correcting or highlighting a bias, e.g., as feedback. In an embodiment, the training set comprises multiple images and the machine learnable model is configured to receive an image as input.

In an embodiment, relationships between decision makers, in particular personal relationships, are identified, and sensor data is tagged with such identified relationships. This may be done in a structured way so that biases in the data can be more effectively identified and addressed. For example, during training information regarding said relationships may be used to include or exclude certain data points to improve the general objectivity of the data. If a negative influence of personal relationships decisions exceeds a threshold, then such data can be excluded from training, e.g., to increase the quality of the data set. Moreover, further action may be prompted, e.g., the system may generate a signal so that appropriate action can be taken, e.g., an intervention.

In an embodiment, a bias detection system may be configured to detect these personal relationships between stakeholders and use this as input in an algorithm to exclude certain aspects so that the new dataset will stay objective and based on facts, with a reduction in unwanted biases.

In an embodiment, additional information is used to detect bias. For example, a geographical location may be made available, e.g., where the first sensor signal and/or second sensor signal was obtained. As with other sources of information, an overrepresentation of a particular location may be ground to reduce this bias. The inventors have found that doctors around the world can select or process first sensor signals, e.g., images, in different ways. Too much data obtained from, say, a particular country, may cause the usual but biased interpretation to be taken over by the model.

For example, the additional information may be a score of the involved participants. For example, indicating the number of years working in this field. A large amount of data from relatively young or relative old persons may be a ground to reduce this bias in the data.

In an embodiment, patient information may also be used for bias detection. For example, life-style information regarding the patient, e.g., smoking/non-smoking may be relevant for detecting bias in a cancer training set.

Such bias detection systems and/or training systems for machine learnable models, e.g., AI systems are implemented on one or more electronic devices, in particular they may be implemented on a computer. The method of bias detection described herein may be applied in a wide range of practical applications. Such practical applications include the detection of bias for its own sake, but also the improvement of a training set for training machine learnable models. The latter will lead to improved classification or data generation systems.

A person skilled in the art will appreciate that the method may be applied to multi-dimensional image data, e.g., to two-dimensional (2D), three-dimensional (3D) or four-dimensional (4D) images, acquired by various acquisition modalities such as, but not limited to, standard X-ray Imaging, Computed Tomography (CT), Magnetic Resonance Imaging (MRI), Ultrasound (US), Positron Emission Tomography (PET), Single Photon Emission Computed Tomography (SPECT), and Nuclear Medicine (NM).

An embodiment of the method may be implemented on a computer as a computer implemented method, or in dedicated hardware, or in a combination of both. Executable code for an embodiment of the method may be stored on a computer program product. Examples of computer program products include memory devices, optical storage devices, integrated circuits, servers, online software, etc. Preferably, the computer program product comprises non-transitory program code stored on a computer readable medium for performing an embodiment of the method when said program product is executed on a computer.

In an embodiment, the computer program comprises computer program code adapted to perform all or part of the steps of an embodiment of the method when the computer program is run on a computer. Preferably, the computer program is embodied on a computer readable medium.

### BRIEF DESCRIPTION OF THE DRAWINGS

Further details, aspects, and embodiments will be described, by way of example, with reference to the drawings. Elements in the figures are illustrated for simplicity and clarity and have not necessarily been drawn to scale. In the Figures, elements which correspond to elements already described may have the same reference numerals. In the drawings,
Figure 1 schematically shows an example of an embodiment of bias-system for detecting bias,
Figure 2a schematically shows an example of an embodiment of bias-system for detecting bias,
Figure 2b schematically shows an example of an embodiment of bias-system for detecting bias,
Figure 3 schematically shows an example of an embodiment of a classifier,
Figure 4 schematically shows an example of an embodiment of a bias detection unit,
Figure 5a schematically shows an example of an embodiment of a method for detecting bias,
Figure 5b schematically shows an example of an embodiment of a method for detecting bias,
Figure 6a schematically shows a computer readable medium having a writable part comprising a computer program according to an embodiment,
Figure 6b schematically shows a representation of a processor system according to an embodiment.

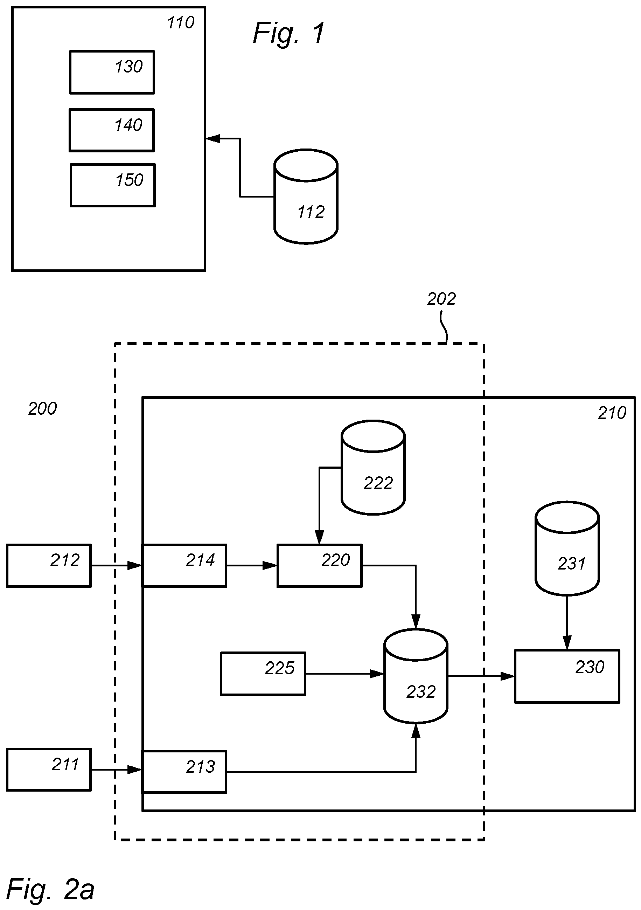

### List of Reference Numerals in figures 1-4, 6a, 6b:

- 110: a bias-device
- 112: a database
- 130: a processor
- 140: a memory
- 150: a communication interface

- 200, 201: a bias system
- 202: a bias detect part
- 210: a bias-device
- 211: a first sensor
- 212: a second sensor
- 213: a first sensor interface
- 214: a second sensor interface
- 222: a template database
- 220: an identification unit
- 225: bias analyzer
- 232: a training database
- 233: additional training data
- 230: a training system
- 231: a parameter database

- 300: a classification system
- 310: a classification device
- 311: a first sensor
- 313: a first sensor interface
- 331: trained parameters
- 330: a machine learnable model
- 340: a display interface
- 400: a bias detection unit
- 410: a clinical knowledge database
- 420: a personnel profile database
- 430: a scoring system
- 440: a transcription system
- 450: a decision system

- 1000: a computer readable medium
- 1010: a writable part
- 1020: a computer program
- 1110: integrated circuit(s)
- 1120: a processing unit
- 1122: a memory
- 1124: a dedicated integrated circuit
- 1126: a communication element
- 1130: an interconnect
- 1140: a processor system

### DETAILED DESCRIPTION OF EMBODIMENTS

While the presently disclosed subject matter is susceptible of embodiment in many different forms, there are shown in the drawings and will herein be described in detail one or more specific embodiments, with the understanding that the present disclosure is to be considered as exemplary of the principles of the presently disclosed subject matter and not intended to limit it to the specific embodiments shown and described.

In the following, for the sake of understanding, elements of embodiments are described in operation. However, it will be apparent that the respective elements are arranged to perform the functions being described as performed by them.

Further, the presently disclosed subject matter is not limited to the embodiments, as feature described herein or recited in mutually different dependent claims may be combined.

**Figure 1** schematically shows an example of an embodiment of bias-system for detecting bias, in this case embodied as a bias-device 110. Bias-device 110 comprises a processor 130, a memory 140 and a communication interface 150. For example, processor 130 may be configured to execute instructions stored in memory 140. Part of memory 140 may be volatile, e.g., RAM and part may be non-volatile, e.g., Flash. Bias-Device may also comprise other types of storage, e.g., a hard-disk, cd-rom, etc.

Bias-device 110 may make use of various collections of data, e.g., one or more databases 112; for example, database 112 may comprise templates of people, e.g., voice templates, parameters of a machine learnable, and so on. Database 112 may be used for storage, e.g., of training data, e.g., de-biased training data, e.g., training associated with biasing-information, and so on. Memory 140 and database 112 may be local or external. For example, all or part of memory 140, and/or of database 112 may be implemented locally or may be implemented externally, e.g., in cloud storage.

Communication interface 150 may comprise a first sensor interface configured to receive multiple first sensor signals obtained through a first sensor, and/or a second sensor interface configured to receive multiple second sensor signals obtained through a second sensor.

Software stored in memory 140 may be configured to identify persons in the second sensor signal, e.g., based on templates. Software stored in memory 140 may be configured to identify a bias in the first sensor signals using the identified persons, e.g., participants in an interaction. The detected bias may be used, e.g., to reduce the bias in the data, or to tag the bias etc. Device 110 may be configured to train a machine learnable model on first sensor data, e.g., a classifier or a data generator, wherein the detected bias is taken into account. For example, bias can be taken into account by appropriately weighing the bias information. For example, bias can be taken into account by excluding part of the biased data from the training.

Bias device 110 may be implemented as a single device, e.g., as shown in figure 1, but may also be implemented in a distributed fashion. For example, processor 130 may comprise multiple microprocessors, which need not all be located at the same physical location. The same holds for the memory 140, which may be cloud-memory, implemented as different locations, etc.

The various parts of device/system 110 may communicate with each other over a computer network. The computer network may be an internet, an intranet, a LAN, a WLAN, etc. The computer network may be the Internet. The computer network may be wholly or partly wired, and/or wholly or partly wireless. For example, the computer network may comprise Ethernet connections. For example, the computer network may comprise wireless connections, such as Wi-Fi, ZigBee, and the like. The parts may comprise a connection interface which is arranged to communicate with other devices of system 110 as needed. For example, the connection interface may comprise a connector, e.g., a wired connector, e.g., an Ethernet connector, an optical connector, etc., or a wireless connector, e.g., an antenna, e.g., a Wi-Fi, 4G or 5G antenna.

In particular the communication between a sensor and device 110, e.g., a sensor interfaced of device 110 may be done over a computer network.

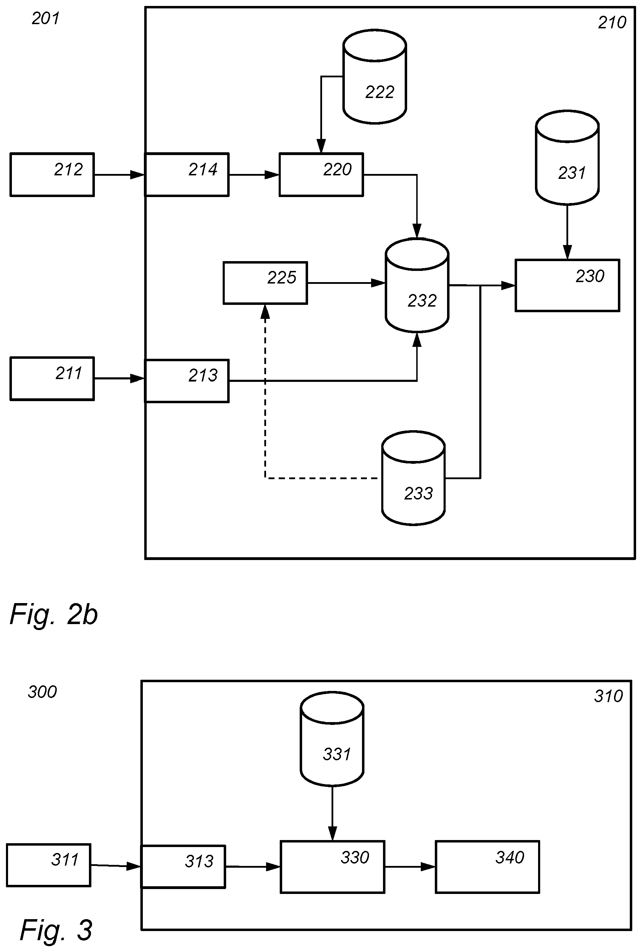

The execution of bias system 110 is implemented in a processor circuit, examples of which are shown herein. Other figures, e.g., figures 2a, 2b and 3 show functional units that may be functional units of a processor circuit. For example, these figures may be used as a blueprint of a possible functional organization of a processor circuit. The processor circuit is not shown separate from the units in those figures. For example, the functional units shown in the figures may be wholly or partially implemented in computer instructions that are stored, e.g., at device 110, e.g., in an electronic memory of device 110, and are executable by a microprocessor of device 110. In hybrid embodiments, functional units are implemented partially in hardware, e.g., as coprocessors, e.g., neural network coprocessors, and partially in software stored and executed on device 110.

**Figure 2a** schematically shows an example of an embodiment of bias-system 200 for detecting bias. Bias-system 200 comprises a first sensor 211 and a second sensor 212, and a bias-device 210. For example, bias-device 210 may be device 110, or the like. Bias-device 210 may be implemented as a distributed system.

Bias-device 210 comprises a first sensor interface 213 configured to receive multiple first sensor signals obtained through first sensor 211. For example, the first sensor signal may comprise an image, e.g., a radiology image, or a pathology slide, etc. For example, device 210 may obtain the first sensor signals from a patient dossier. For example, an image sensor may upload an image to a patient file from which it may become available to device 210. Typically, at least part of the first sensor signal may later be used for training a machine learnable model. However, there may be a bias in the data obtained through the first sensor.

Unfortunately, directly using the first sensor signals for training may not be ideal, since the data may be biased. For example, some aspects in the data may be overrepresented, or underrepresented, etc. Some of the data may be wrong. Some of this bias may be reduced using information obtained from second sensor 212.

Bias-device 210 comprises a second sensor interface 214 configured to receive multiple second sensor signals obtained through second sensor 212. For example, part of the second sensor signals comprise an interaction between at least two or more participants in an environment around the second sensor. For example, the second sensor signal may comprise information on people, e.g., participants, discussing the first sensor signal. For example, the second sensor signal may be video or an image or images of said persons. In an embodiment, the second signal is an audio signal, e.g., a recording of people discussing a first sensor signal. For example, in an embodiment, the first sensor signal may comprise an image, e.g., a radiology image, and the second sensor signal may comprise an audio recording of people, e.g., medical personnel, discussing the image.

Device 210 may comprise an identification unit 220 and a template database 222. For example, template database 222 may comprise template data configured to identify a participant. Identification unit 220, e.g., an identification system, may be configured to identify one or more of the participants in the second sensor signal by matching a participant with a corresponding template. The template may be a visual template, e.g., biometric template. The template may be used to recognize a person from an image. For example, identification unit 220 may be configured for facial recognition.

In an embodiment, template database 222 comprises audio templates, also known as voice prints. Identification unit 220 may be configured for speaker recognition. There are numerous known ways to derive a template for a person and to use it to recognize a speaker. For example, technologies used to process and store voice prints may include frequency estimation, hidden Markov models, Gaussian mixture models, pattern matching algorithms, neural networks, matrix representation, vector quantization and decision trees, etc. Further details are discussed in "Local spectral variability features for speaker verification", by Sahidullah, Md. Kinnunen, Tomi., which proposes to perform speaker verification by characterizing the spectral variations of a speaker. Another implementation can be found in "Application of MFCC in Text Independent Speaker Recognition" by Shipra Gupta, which proposes to extract Mel Frequency Cepstrum Coefficient (MFCC) features for speaker identification.

For example, the system 210 may be configured for two phases: Enrollment and identification. During enrollment, a template is created, e.g., a speaker's voice is recorded, a number of features are extracted to form a voice print, or template, or an image is taken, and a facial recognition template is crated. In the identification phase, say, a speech sample or "utterance" is compared against a previously created voice print. For identification systems, the utterance may be compared against multiple voice prints in database 222 in order to determine the best match or matches. Enrollment maybe implicit. For example, when a participant is not recognized, e.g., cannot be matched with a template, then a template may be created to recognize the participant from in future.

For example, in an embodiment, the first sensor signals may comprise medical data pertaining to a patient. For example, a first sensor signal may comprise an image, e.g., a radiology image, a pathology slide, etc. For example, in an embodiment, the second sensor signals may comprise an audio recording, e.g., of a tumorboard discussing the first sensor signal, or part thereof, e.g., the radiology image. The second sensor signal could also or instead comprise an image, e.g., of the tumorboard discussing the first sensor signal
For example, identification system 220 may be configured to tag a first and/or second sensor signal with a label indicating a participant detected in a second sensor signal. System 210 may receive a first sensor signal and a corresponding second sensor signal. Identification system 220 may identify in the second sensor signal one or more participants to a discussion pertaining to the first sensor signal. For example, the second sensor signal may be compared to one or more templates, e.g., facial or voice templates that are stored in template database 222. Accordingly, identification system 220 may tag the first sensor signal with one or more tags that indicate the detected participants.

In an embodiment, the identification system is configured to identify further properties in the second sensor signal. For example, the identification system may be configured to identify a group dynamic between the at least two participants. For example, the two participants may disagree, of agree, may be sympathetic or have an argument and so on. This may be done both from audio and from imagery. For example, volume may be raised in case of disagreement. For example, facial expression may be recognized that signal agreement or non-agreement. The first sensor data may be tagged with the detected group dynamic. Faces and group dynamics may be recognized from an image using a neural network, e.g., a deep neural network. Bias detection may be solely from a detected group dynamic as well.

The tagged first sensor signals may be stored in a training database 232. Tags may be applied to a file representing a first sensor signal. But tags may also be kept in a separate file, e.g., pointing to the relevant first sensor signal. Instead of storing tags, they may instead by used directly to detect bias and, e.g., to exclude the corresponding first sensor signal from database 232.

System 210 may further comprise a bias analyzer 225 or bias detection system. Bias analyzer 225 is configured to identify a bias in the multiple first sensor signals using the identified participants. For example, bias analyzer 225 may be configured to detect a bias in the multiple first sensor signals if a tag or a combination of tags is over-represented or under-represented.

For example, in an embodiment, the bias analyzer 225 is configured to count the number of first sensor signal tagged with a particular participant. If the number of first sensor signal so tagged is above a threshold, e.g., an absolute or relative threshold, a bias may be detected. For example, if a combination of multiple tags, in particular two tags, occurs more than a threshold, e.g., more than 100 signals or more than 10% of the signals, etc., a bias may be detected. The tags may refer to participants but also to group dynamics. For example, a bias may be present if too many first signals have a particular participant and as group dynamic disagreement. For example, a bias may be present if too many first signals have two particular participants and the group dynamic agreement, and so on.

For example, if a large part of the data is associated with the person of the same set of people, e.g., the same two medical professionals, etc., then a bias that these persons may have, may be reflected in the data. Thus, there is a risk that this bias will also be reflected in a trained model. By limiting the amount or the weight of data, if too much of it comes from the same person or set of persons, this risk is reduced.

First sensor interface 213, second sensor interface 214, identification unit 220, template database 222, bias analyzer 225 and training database 232 may together be referred to as a bias detect part 202.

Detected bias may be recorded, e.g., stored in a file, it may be reported, e.g., to an operator of system 210.

System 210 may further comprise a training system 230. Training system 230 is coupled to a parameter database 231. For example, parameter database 231 may be configured to comprise multiple parameters representing a machine learnable model. Training system 230 may be configured for training the machine learnable model by repeatedly applying the machine learnable model to a first sensor signal and updating the multiple parameters in response thereto. For example, the machine learnable model may be a conventional type of machine learnable model, e.g., a support vector machine (SVM), random forests, neural network and the like.

Interestingly, training system 230 may be configured to take into account the bias that is detected by bias analyzer 225. For example, training system 230 may be configured to adapt the weight of a first sensor signals in dependence on detected bias in an associated second sensor signal. For example, training system 230 may be configured with a learning rate. The learning rate indicates how strongly the parameters of parameter database 231 are changed in response to a wrong prediction of the machine learnable model. For example, the parameters may be modified in dependence one or more of the learning rate, a derivate of the model with the respect to a current input and parameter and the size of the current error; for example, the may be modified from a multiplication of the learning rate and the derivate and the size of the error; for example, a learning algorithm such as backpropagation may be used. Backpropagation learning algorithms may be applied to a variety of machine learnable models, in particular to neural networks. For example, a default learning parameter λ₁ may be used, e.g., for most first sensor signals; however, for first sensor signals with a detected bias a learning parameter, e.g., rate, of λ₂ may be used. One may use λ₁ > λ₂, e.g., λ₁ ≥ 2λ₂.

In an embodiment, a weight *p* is determined for first sensor signals. The higher the value of *p* the stronger the bias. For example, when training on that first signal, the learning parameter may be taken as *λf*(*p*), wherein *p* is a decreasing function, e.g., *λ*/*p*, etc. For example, the weight p and/or the corresponding learning rate, may be capped, from above or below, e.g., cut-off at an upper-threshold and/or lower-threshold. The weight p or the corresponding learning rate may be binned; for example, first signals may be portioned in high, low and medium bias, each with their own learning rate. If the bias is under-representation, then the learning-rate may be increased, e.g., increased with respect to a default.

Instead of adapting the learning rate, one may also directly change the bias, by excluding some first sensor signals. For example, if a particular tag occurs very often, then part of them may be excluded, e.g., a random selection. These methods can also be combined, e.g., adapting learning rate for some signals, e.g., low bias signals, and excluding other signals, e.g., high bias signals.

For example, suppose that in a set of 100 first signals, 60 come from the same doctor. There is thus a risk that the doctor's biases will be reflected in the trained model. This may be addressed by removing, say, 30 of the 60 first signals from that doctor. This will improve the balance between the various contributors to the data. On the other hand, the full set of 60 signals may be used, but with a lower learning rate. The latter has the advantage that the model is still exposed to all the available data. The two options can be combined, say, remove 10 signals, say at random, or selected on the basis of a further criterion, and remove the learning rate slightly. One could also increase the learning rate for the 40 non-biased signals.

**Figure 2b** schematically shows an example of an embodiment of bias-system 201 for detecting bias. The system of figure 2b is similar to that of figure 2a except that a source of additional training data 233 is used. For example, additional training data may be a patient parameter associated with the patient that corresponds to the first sensor signal. For example, the patient parameter may be a medical parameter that the model is to predict from the first sensor data. For example, the patient parameter may be values such as age, weight, blood pressure. The additional parameter may also be an interpretation of an image in a first signal, e.g., medical segmentations. For example, the patient parameter may, e.g., comprise a clinical outcome.

Optionally, the additional information may be used to detect bias. For example, a database with clinical knowledge may be used to tag first data which seems unusual. For example, very long or short hospital stay, an unusual progression of an illness. Having a proportion of data which in a training set than what is known to be statistically likely may reduce the correctness of the model. For example, if a treatment typically takes a year, then having many data points in which it is only a month, may-even if this data is correct in itself-cause bias. For example, information may be used regarding the patients, e.g., lifestyle, or the participants, e.g., experience, may be used. For example, a scoring system may be configured to retrospectively detect anomalous decisions based on the first sensor data, and to assign a performance score for a participant. A performance score may also or instead be based on the number of years of experience or the number of cases treated, etc. Lifestyle information is relevant since it may be the case that some lifestyle choices, e.g., smoking, since knowing that someone smoked may of itself lead to a higher probability that an image is interpreted as lung cancer.

Information may also be added about the role of a person in an organization. The particular role, e.g., assistant or doctor, may be used to detect a bias in the data.

For example, a geographical location may be made available, e.g., where the first sensor signal and/or second sensor signal was obtained. As with other sources of information, an overrepresentation of a particular location may be ground to reduce this bias. The inventors have found that doctors around the world can select or process first sensor signals, e.g., images, in different ways. Too much data obtained from, say, a particular country, may cause the usual but biased interpretation to be taken over by the model.

For example, the information may include the background of the patient and/or of staff. For example, where the patient or reviewees come from, etc. This can be relevant since staff may be trained differently around the world.

For example, the additional information may be a score of the involved participants. For example, indicating the number of years working in this field. A large amount of data from relatively young or relative old persons may be a ground to reduce this bias in the data.

In an embodiment, patient information may also be used for bias detection. For example, lifestyle information regarding the patient, e.g., smoking/non-smoking may be relevant for detecting bias in a cancer training set.

In an embodiment, notes taken by participant may be analyzed as well, e.g., information not obtained from a sensor. For example, the notes may indicate the participants.

In an embodiment of a bias detection system, e.g., system 210 in figure 2a or 2b, the bias detection part, e.g., bias analyzer 225 may comprise a rule-system configured for multiple rules for detecting bias. For example, the multiple rules may comprise a rule indicating that a high occurrence of any tag is regarded as bias. The multiple rules may comprise a rule indicating that a high occurrence of person-tag is regarded as bias, but a high occurrence of a particular illness is not. Many types of rules may be formulated, as indicated herein, e.g., depending on experience, location, participants, group dynamic, and so on. In an embodiment, the rules are downloadable. For example, a rule set may be expressed in XML or other computer parsable language. This has the advantage that rules can be exchanged.

There are many machine learnable models that may be trained on the first sensor signal. For example, one may learn a classifier on the first sensor signal. A classifier may be supervised or unsupervised. For example, a classifier may be trained on first signals comprising radiology images together with further data which indicates if the images correspond to a cancer. The classifier will thus learn to classify images as corresponding to a cancer or to a non-cancer. Interestingly, if in the collection of first signals many signals happen to correspond to a particular practitioner, then these signals may be given less weight in the training, to avoid that these images will dominate the training of the model. Bias may not be apparent to a human but may be picked up by an algorithm.

Another example, of a machine learnable model is a data generator; for example, a generative adversarial model (GAN). In this case, the objective may be to generate additional images that have a similar distribution. For example, if the first signals comprise radiology images, then the objective of the GAN may be to generate images that look just like such radiology images. Having a model that can generate additional examples of the type of images under interest is very helpful in developing medical systems. For example, the additional images generated by the model, may be used for testing other medical systems and for testing or training other models. There is only a finite amount of training material available, and it is difficult to acquire much more. Apart from the time and cost to obtain additional images, there are only a limited number of patients, and they cannot be subjected to much more imaging procedures than are needed for their treatment.

**Figure 3** schematically shows an example of an embodiment of a classifier system 300. Classifier system 300 comprises a classifier device 310 and a first sensor 311. First sensor 311 may be of the same kind as first sensor 211, though not necessarily, the same sensor. Classifier device 310 may comprise a first sensor interface 313 configured to receive a first sensor signal from first sensor 311. For example, a first sensor signal may comprise an image, etc., as indicated above for signal 211. Device 310 may comprise a set of trained parameters 331 for a machine learnable model 330. The trained parameters may have been obtained with a system such as system 200 or 201.

In this case, the model is a classifier and the output of the model is some prediction about a first sensor signal, e.g., a patient parameter, such as a clinical outcome. The result of the prediction may be used by a further part of device 310. For example, the device may comprise an optional display interface 340 configured to display the result of model 330 on a display. For example, the model may be used to sort the set of first sensor signal in an order of likely more serious to less serious, so that a trained practitioner may evaluate the signals in an order ranging from more to likely less serious.

For example, classifier 300 may comprise a U-net and trained to segment the first sensor signals according to a medically relevant criterion, e.g., outline of a tumor, outline of an intestine, and so on. Ground truth for training may be provided in database 233.

In an embodiment, system 300 may be configured to give information regarding the level of bias found int the training data, so that a user of system 300 can better gauge the correctness of the data.

In the various embodiments of bias detect or classification or data generation device, etc., a communication interface may be included which may be selected from various alternatives. For example, the interface may be a network interface to a local or wide area network, e.g., the Internet, a storage interface to an internal or external data storage, a keyboard, an application interface (API), etc.

These devices may have a user interface, which may include well-known elements such as one or more buttons, a keyboard, display, touch screen, etc. The user interface may be arranged for accommodating user interaction for performing a bias detection, a training, a classification, a data generation, and so on.

These devices may comprise storage which may be implemented as an electronic memory, say a flash memory, or magnetic memory, say hard disk or the like, or optical memory, e.g., a DVD. Storage may comprise multiple discrete memories together making up the storage. Storage may comprise a temporary memory, say a RAM

Typically, these devices each comprise a microprocessor which executes appropriate software stored at the device.; for example, that software may have been downloaded and/or stored in a corresponding memory, e.g., a volatile memory such as RAM or a non-volatile memory such as Flash. The devices may also be equipped with microprocessors and memories. Alternatively, the devices may, in whole or in part, be implemented in programmable logic, e.g., as field-programmable gate array (FPGA). The devices may be implemented, in whole or in part, as a so-called application-specific integrated circuit (ASIC), e.g., an integrated circuit (IC) customized for their particular use. For example, the circuits may be implemented in CMOS, e.g., using a hardware description language such as Verilog, VHDL, etc.

In an embodiment, the device may comprise circuits such as a processor circuit and storage circuit, the processor circuit executing instructions represented electronically in the storage circuits.

A processor circuit may be implemented in a distributed fashion, e.g., as multiple sub-processor circuits. A storage may be distributed over multiple distributed sub-storages. Part or all of the memory may be an electronic memory, magnetic memory, etc. For example, the storage may have volatile and a non-volatile part. Part of the storage may be read-only.

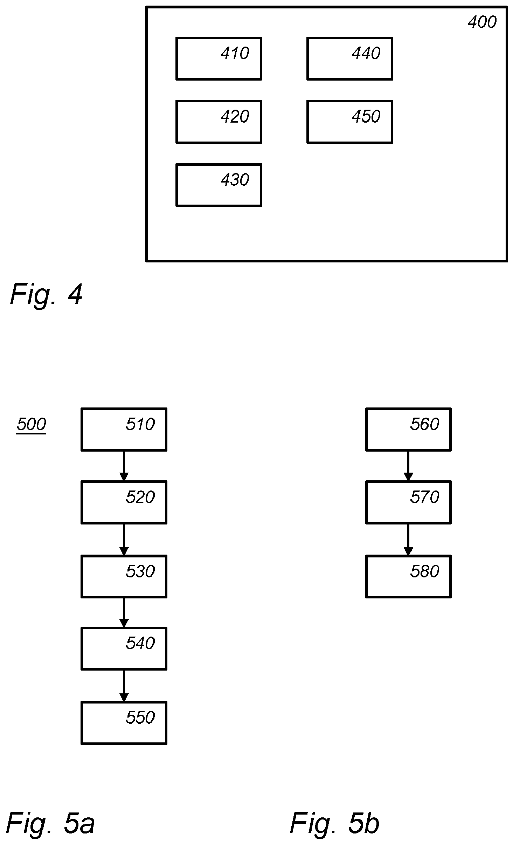

**Figure 4** schematically shows an example of an embodiment of a bias detection unit 400. Bias detection unit 400 may be used in the bias systems as described herein, e.g., in system 200 or 201. For example, bias detection unit 400 may be implemented on a computer; e.g., a processor system may be configured for bias detection unit 400. The bias detection unit may be configured to have access to the first and second sensor signals, etc., as described herein. However, interestingly, unit 400 may also comprise one or more of the following
- A clinical knowledge database 410 (system 1), e.g., comprising a clinical knowledge graph. This may be a system containing clinical knowledge, e.g., based on previous patient cases, hospital protocols and guidelines.
- A personnel profile database 420, e.g., comprising a personal profile graph (system 2). This may be a system that contains personnel profiling data of the decision makers. An example could be organizational data, e.g., such as an organigram, that maps out the organization hierarchy. It may also contain detailed profile information about the stakeholders, e.g., explaining their background (e.g. CV, data about their performance in their current role, social media data and relationships, background information).
- A scoring system 430 (system 2). This may be a system that retrospectively detects any anomalies or wrong clinical decisions made in previous cases/meetings by each stakeholder. It may use rules, e.g., from the clinical knowledge graph, to detect if certain decisions were not correct looking at the patient case outcome. Based on this, a performance score can be calculated for each stakeholder.
- A transcription system 440. This may be a system that transcribes, e.g., in real time, a meeting, such as, a multi-disciplinary meeting, e.g., using NLP technologies to understand the information that is discussed. It may automatically detect who is saying what, and store this in a structured format.
- A decision system 450. This system may detect, e.g., based on the information coming out of the transcription system, which participant is saying what, and would hold any conclusions or hypothesis against the clinical knowledge graph.

For example, if any conclusions, actions or other decisions are proposed by one or more stakeholders, e.g., participants, the system may check these against the clinical knowledge database (system 1). If an action or conclusion is not in line with, e.g., a clinical guideline or protocol, the system may further analyze who proposed this specific action. Further analysis may use profiling data (System 2) to map out the background of the stakeholder, e.g., role, experience, previous cases, to determine if there is a reason to doubt this conclusion. The system may also be configured to detect, e.g., with sentiment analysis, what the tone of voice is during the meeting, and use this information to further define if there was a specific bias in place. In case there was a heated discussion and many disagreements between peers, this information can then be used to further exclude biases or add a remark to the labelled data. The system may also be configured to detect body language. For example, the system may comprise an image sensor, e.g., for video or still images, or an interface thereto. The system may be configured to analyze the image data for body language and annotate the first sensor signal with detected body language.

The following example scenario describes how this system may be used:
- The weekly lung-tumorboard comes together. Today's agenda includes around 25 patient cases that are waiting for a definitive medical conclusion and treatment plan.
- Part of the committee is the chairman who controls the PACS system, and the tumorboard information system. Next to him, a radiologist, a pathologist, social workers, and several surgeons are attending this meeting, all with their own specialties and domain knowledge.
- The tumorboard is opened, and in the background the system automatically starts to transcribe everything that is being said in the room.
- The first patient is discussed, and radiology images are shown and pathology slides are selected as well. The chairman asks the radiologist what he concludes out of the images. He mentions that he is almost certain this is about a particular lung tumor.
- The system transcribes this and understands that this comment is a conclusion of the radiologist. The system then looks into the patient case, and checks if the remarks are in line with clinical protocols. It also checks the profile of the radiologist and based on his track record the system scores this conclusion with a high rate.
- The team moves on to the bi-opt part and the pathologist also agrees with the earlier statement: based on the genomic factors of the tumor cells, this is indeed a lung tumor. The system also checks this with the previous mentioned systems to check this hypothesis and agrees with the statement.
- In the tumor board information system (that also shows the notes that are taken by the system) visually shows the score of the conclusions for all attendees to see.
- The last part of the patient case discussion is about the follow-up steps and treatment selection.
- The radiologists and surgeon do not fully agree about what the best treatment option will be. The radiologist would like to refer the patient to a university hospital, where the surgeon states that treating this patient in-hospital will the better option.
- The system recognizes that there is a conflict and tags this in the data.
- The chairman concludes that treating the patient in the hospital would be the best option for this particular patient and rounds off. They move on to the next patient.
- The system uses the clinical protocols, previous patient cases, and other information to do further analysis. It finds out that the surgeon reports to the chairman in the organigram, and that they made a similar decision in the past. The result here was that the patient had to stay longer in the hospital than participated. It seems that the correct clinical protocols are not followed.
- Based on this information, the system decides to tag this patient case and the conclusion as potentially biased, and further follow-up is required in case this data will be used as training data.

**Figure 5a** schematically shows an example of an embodiment of method for detecting method 500. Method 500 is a computer implemented method for detecting bias in information from a sensor system. For example, method 500 may comprise the following:
- receiving (510) multiple first sensor signals obtained through a first sensor,
- receiving (520) multiple second sensor signals obtained through a second sensor, a plurality of the second sensor signals comprise an interaction between at least two or more participants in an environment, a second sensor signal being associated with a first sensor signal,
- providing (530) a template database comprising template data configured to identify a participant,
- identifying (540) one or more of the participants in a second sensor signal by matching a participant with a corresponding template,
- identifying (550) a bias in the multiple first sensor signals using the identified participants.

**Figure 5b** schematically shows an example of an embodiment of method for detecting method. For example, the method parts that are shown in figure 5b may be incorporated in the method shown in figure 5a. For example, method 500 may further comprise:
- providing (560) multiple parameters representing a machine learnable model,
- training (570) the machine learnable model by repeatedly applying the machine learnable model to a first sensor signal and updating the multiple parameters in response thereto, and
- adapting (580) weight of a first sensor signals in dependence on detected bias in an associated second sensor signal, or excluding a first sensor signal with a detected high bias in an associated second sensor signal.

Many different ways of executing the method are possible, as will be apparent to a person skilled in the art. For example, the steps can be performed in the shown order, but the order of the steps may also be varied, or some steps may be executed in parallel. Moreover, in between steps other method steps may be inserted. The inserted steps may represent refinements of the method such as described herein or may be unrelated to the method. For example, some steps may be executed, at least partially, in parallel. Moreover, a given step may not have finished completely before a next step is started.

Embodiments of the method may be executed using software, which comprises instructions for causing a processor system to perform method 500. Software may only include those steps taken by a particular sub-entity of the system. The software may be stored in a suitable storage medium, such as a hard disk, a floppy, a memory, an optical disc, etc. The software may be sent as a signal along a wire, or wireless, or using a data network, e.g., the Internet. The software may be made available for download and/or for remote usage on a server. Embodiments of the method may be executed using a bitstream arranged to configure programmable logic, e.g., a field-programmable gate array (FPGA), to perform the method.

It will be appreciated that the presently disclosed subject matter also extends to computer programs, particularly computer programs on or in a carrier, adapted for putting the presently disclosed subject matter into practice. The program may be in the form of source code, object code, a code intermediate source, and object code such as partially compiled form, or in any other form suitable for use in the implementation of an embodiment of the method. An embodiment relating to a computer program product comprises computer executable instructions corresponding to each of the processing steps of at least one of the methods set forth. These instructions may be subdivided into subroutines and/or be stored in one or more files that may be linked statically or dynamically. Another embodiment relating to a computer program product comprises computer executable instructions corresponding to each of the devices, units and/or parts of at least one of the systems and/or products set forth.

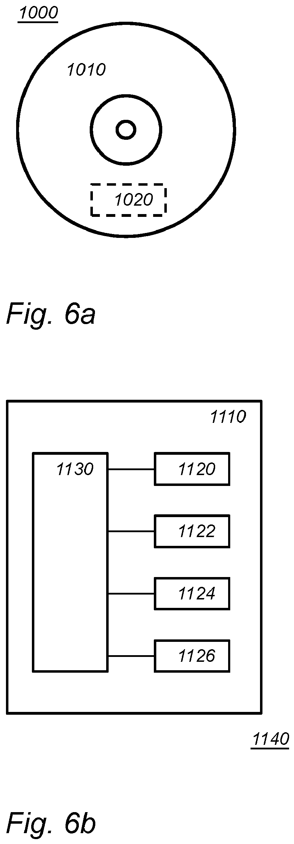

**Figure 6a** shows a computer readable medium 1000 having a writable part 1010 comprising a computer program 1020, the computer program 1020 comprising instructions for causing a processor system to perform a method for bias detection according to an embodiment. The computer program 1020 may be embodied on the computer readable medium 1000 as physical marks or by magnetization of the computer readable medium 1000. However, any other suitable embodiment is conceivable as well. Furthermore, it will be appreciated that, although the computer readable medium 1000 is shown here as an optical disc, the computer readable medium 1000 may be any suitable computer readable medium, such as a hard disk, solid state memory, flash memory, etc., and may be non-recordable or recordable. The computer program 1020 comprises instructions for causing a processor system to perform said method of bias detection.

**Figure 6b** shows in a schematic representation of a processor system 1140 according to an embodiment of a bias detection system and/or of a trained machine learnable model. The processor system comprises one or more integrated circuits 1110. The architecture of the one or more integrated circuits 1110 is schematically shown in Figure 6b. Circuit 1110 comprises a processing unit 1120, e.g., a CPU, for running computer program components to execute a method according to an embodiment and/or implement its modules or units. Circuit 1110 comprises a memory 1122 for storing programming code, data, etc. Part of memory 1122 may be read-only. Circuit 1110 may comprise a communication element 1126, e.g., an antenna, connectors or both, and the like. Circuit 1110 may comprise a dedicated integrated circuit 1124 for performing part or all of the processing defined in the method. Processor 1120, memory 1122, dedicated IC 1124 and communication element 1126 may be connected to each other via an interconnect 1130, say a bus. The processor system 1110 may be arranged for contact and/or contact-less communication, using an antenna and/or connectors, respectively.

For example, in an embodiment, processor system 1140, e.g., the bias detection device may comprise a processor circuit and a memory circuit, the processor being arranged to execute software stored in the memory circuit. For example, the processor circuit may be an Intel Core i7 processor, ARM Cortex-R8, etc. In an embodiment, the processor circuit may be ARM Cortex M0. The memory circuit may be an ROM circuit, or a non-volatile memory, e.g., a flash memory. The memory circuit may be a volatile memory, e.g., an SRAM memory. In the latter case, the device may comprise a non-volatile software interface, e.g., a hard drive, a network interface, etc., arranged for providing the software.

As used herein, the term "non-transitory" will be understood to exclude transitory signals but to include all forms of storage, including both volatile and non-volatile memories.

While device 1100 is shown as including one of each described component, the various components may be duplicated in various embodiments. For example, the processor 1120 may include multiple microprocessors that are configured to independently execute the methods described herein or are configured to perform steps or subroutines of the methods described herein such that the multiple processors cooperate to achieve the functionality described herein. Further, where the device 1100 is implemented in a cloud computing system, the various hardware components may belong to separate physical systems. For example, the processor 1120 may include a first processor in a first server and a second processor in a second server.

It should be noted that the above-mentioned embodiments illustrate rather than limit the presently disclosed subject matter, and that those skilled in the art will be able to design many alternative embodiments.

In the claims, any reference signs placed between parentheses shall not be construed as limiting the claim. Use of the verb 'comprise' and its conjugations does not exclude the presence of elements or steps other than those stated in a claim. The article 'a' or 'an' preceding an element does not exclude the presence of a plurality of such elements. Expressions such as "at least one of' when preceding a list of elements represent a selection of all or of any subset of elements from the list. For example, the expression, "at least one of A, B, and C" should be understood as including only A, only B, only C, both A and B, both A and C, both B and C, or all of A, B, and C. The presently disclosed subject matter may be implemented by hardware comprising several distinct elements, and by a suitably programmed computer. In the device claim enumerating several parts, several of these parts may be embodied by one and the same item of hardware. The mere fact that certain measures are recited in mutually different dependent claims does not indicate that a combination of these measures cannot be used to advantage.

In the claims references in parentheses refer to reference signs in drawings of exemplifying embodiments or to formulas of embodiments, thus increasing the intelligibility of the claim. These references shall not be construed as limiting the claim.

## Claims

1. A bias-system for detecting bias in information from a sensor system, the bias-system comprising
- a first sensor interface configured to receive multiple first sensor signals obtained through a first sensor,
- a second sensor interface configured to receive multiple second sensor signals obtained through a second sensor, a plurality of the second sensor signals comprise an interaction between at least two or more participants in an environment, a second sensor signal being associated with a first sensor signal,
- a template database comprising template data configured to identify a participant,
- an identification system configured to identify one or more of the participants in a second sensor signal by matching a participant with a corresponding template,
- a bias detection system configured to identify a bias in the multiple first sensor signals using the identified participants.

2. A system as in claim 1, comprising
- a parameter database configured to comprise multiple parameters representing a machine learnable model,
- a training system for training the machine learnable model by repeatedly applying the machine learnable model to a first sensor signal and updating the multiple parameters in response thereto, wherein
- a weight of a first sensor signals is adapted in dependence on detected bias in an associated second sensor signal, or wherein a first sensor signal is excluded with a detected high bias in an associated second sensor signal.

3. A system as in any one of the preceding claims, wherein
- a first sensor signal comprises an image, e.g., a radiology image, or a pathology slide, and/or
- a second sensor signal comprises an audio signal and/or an image.

4. A system as in any of the preceding claims wherein,
- the identification system is configured to tag a first and/or second sensor signal with a label indicating a participant detected in a second sensor signal.

5. A system as in claim 4 wherein, the bias detection system is configured to identify a bias in the multiple first sensor signals if a tag or a combination of tags is over-represented or under-represented.

6. A system as in any of the preceding claims wherein the identification system is configured to identify a group dynamic between the at least two participants, and to tag the first and/or second sensor data with a label indicating the identified group dynamic.

7. A system as in any of the preceding claims wherein the first sensor data is provided with a patient parameter, e.g., a clinical outcome.

8. A system as in any of the preceding claims wherein the first sensor data is provided with geographical information where a first and/or second sensor signal was obtained, the bias detection system being configured to process the geographic information.

9. A system as in any one of the preceding claims, comprising
- a clinical knowledge database comprising clinical knowledge, and/or
- a personnel profile database comprising a personal profile of participants, and/or
- a scoring system configured to retrospectively detect anomalous decisions based on the first sensor data, and to assign a performance score for a participant.

10. A machine learnable model trained by a system according to claim 2 and optionally any other one of the preceding claims, wherein
- the machine learnable model comprises a classifier configured to receive an image and to generate a classification of the image, and/or
- the machine learnable model comprises a data generator configured to generate an image according to an input of the machine learnable model.

11. A computer implemented method (500) for detecting bias in information from a sensor system, the method comprising
- receiving (510) multiple first sensor signals obtained through a first sensor,
- receiving (520) multiple second sensor signals obtained through a second sensor, a plurality of the second sensor signals comprise an interaction between at least two or more participants in an environment, a second sensor signal being associated with a first sensor signal,
- providing (530) a template database comprising template data configured to identify a participant,
- identifying (540) one or more of the participants in a second sensor signal by matching a participant with a corresponding template,
- identifying (550) a bias in the multiple first sensor signals using the identified participants.

12. A method as in claim 11, comprising
- providing (560) multiple parameters representing a machine learnable model,
- training (570) the machine learnable model by repeatedly applying the machine learnable model to a first sensor signal and updating the multiple parameters in response thereto, and
- adapting (580) weight of a first sensor signals in dependence on detected bias in an associated second sensor signal, or excluding a first sensor signal with a detected high bias in an associated second sensor signal.

13. A transitory or non-transitory computer readable medium (1000) comprising data (1020) representing instructions, which when executed by a processor system, cause the processor system to perform the method according to claim 11 or 12.
